# EUROPÄISCHE PATENTSCHRIFT

(11) **EP 2 252 227 B1**
(45) Veröffentlichungstag und Bekanntmachung des Hinweises auf die Patenterteilung: **10.10.2012**
(21) Anmeldenummer: 08716196.4
(22) Anmeldetag: 03.03.2008
(51) Int. Cl.: A61B 18/12, A61B 18/16

(54) **VORRICHTUNG FÜR DIE THERMOCHIRURGIE**
DEVICE FOR THERMOSURGERY
DISPOSITIF DE THERMOCHIRURGIE

(43) Veröffentlichungstag der Anmeldung: 24.11.2010
(73) Patentinhaber: Stockert GmbH, 79111 Freiburg (DE)
(72) Erfinder: Stockert, Rüdiger, 79104 Freiburg (DE)
(74) Vertreter: Katérle, Axel
(86) Internationale Anmeldenummer: PCT/EP2008/001677
(87) Internationale Veröffentlichungsnummer: WO 2009/109197

(56) Entgegenhaltungen:
- EP-A- 1 808 144
- WO-A-2005/110263
- US-A1- 2004 059 323

## Beschreibung

Die Erfindung betrifft eine Vorrichtung für die Thermochirurgie.

Bei der Thermochirurgie wird biologisches Gewebe durch Einbringung von Behandlungsenergie erwärmt, um durch Denaturierung des behandelten Gewebes einen bestimmten therapeutischen Zweck zu erzielen. Insbesondere wird bei der Thermochirurgie eine Koagulation oder Ablation lokaler Gewebebereiche angestrebt, beispielsweise an der inneren Herzwand der Herzkammer, an den Herzkammerventilen, an den mit dem Herz verbunden Venen und Arterien oder anderen blutführenden Gefäßen des menschlichen oder tierischen Körpers. Am Herzen können mittels Thermochirurgie beispielsweise Arrhythmien oder Tachikardien behandelt werden. Es versteht sich, dass das Anwendungsgebiet der erfindungsgemäBen thermochirurgischen Vorrichtung nicht auf Herzbehandlungen beschränkt ist. Prinzipiell eignet sich die erfindungsgemäße Vorrichtung für die thermochirurgische Behandlung beliebiger Stellen am oder innerhalb des Körpers.

Bei der elektrischen HF-Thermochirurgie sind monopolare und bipolare Anwendungen bekannt. Bei beiden Behandlungsarten wird der zur Behandlung dienende hochfrequente Wechselstrom über eine Anwendungselektrode in den Körper eingespeist, die in unmittelbare Nähe des zu behandelnden Gewebebereichs gebracht wird. Bei der monopolaren Technik wird der Stromkreis über eine oder mehrere flächige Gegenelektroden geschlossen, die in weitem Abstand von der Anwendungselektrode außen auf die Haut des Körpers aufgelegt werden. Die wirksame Elektrodenfläche der Gegenelektroden ist groß im Vergleich zu derjenigen der Anwendungselektrode, weshalb unter normalen Umständen (d.h. gutem Kontakt der Gegenelektroden mit der Haut) die Stromdichte an den Gegenelektroden gering ist und dort keine Verkohlung der Haut zu befürchten ist

Die Situation ändert sich, wenn sich die Gegenelektroden teilweise von der Haut ablösen. Dann nimmt die Stromdichte in den noch mit der Haut in Kontakt befindlichen Bereichen der Gegenelektroden stark zu, und dementsprechend größer wird die Gefahr von Hautverkohlungen. Bei thermochirurgischen Behandlungen kann es ohne weiteres vorkommen, dass sich die Behandlungsdauer über mehrere Stunden erstreckt. Bewegungen des Patienten und Schweißbildung können dabei die Kontaktqualität zwischen den Gegenelektroden und der Haut verschlechtern. Mit zunehmender Behandlungsdauer besteht erfahrungsgemäß ein größer werdendes Risiko, dass der elektrische Kontakt zwischen den Gegenelektroden und der Haut schlechter wird.

Aufgabe der Erfindung ist es, eine Vorrichtung für die Thermochirurgie bereitzustellen, die zuverlässig unerwünschte Gewebe- bzw. Hautverbrennungen vermeiden kann.

Zur Lösung dieser Aufgabe sieht die Erfindung eine Vorrichtung gemäß Anspruch 1 ror.

Dokument US-A-2004/059323 offenbart eine Vorrichtung gemäß Oberbegrift des Anspruchs 1

Die erfindungsgemäße Vorrichtung gestattet die Ermittlung mehrerer Impedanzwerte mit unterschiedlicher Aussagekraft, wobei in jeden Impedanzwert die Gewebeimpedanz eines jeweils anderen Teils des Körpers des Patienten einfließt. Die vereinigte Aussagekraft der mehreren gemessenen Impedanzwerte kann dann eine zuverlässige Bestimmung ermöglichen, an welcher Gegenelektrode es Kontaktprobleme beispielsweise aufgrund teilweiser Ablösung der Elektrode gibt.

Es sind vielfältige Konfigurationen von Messkreisen vorstellbar, mit denen sich insgesamt eine Mehrzahl verschiedener Impedanzen messen lassen. So kann gemäß einer Weiterbildung der Erfindung die Schnittstellenanschlussanordnung den Anschluss mehrerer Gegenelektrodeneinheiten mit je zwei Gegenelektroden gestatten, wobei die Messkreise in Zuordnung zu jeder Gegenelektrodeneinheit je einen ersten Messkreis umfassen, in dem die Gegenelektroden der betreffenden Gegenelektrodeneinheit elektrisch in Reihe liegen.

Alternativ oder zusätzlich kann die Schnittstellenanschlussanordnung den Anschluss mindestens einer Gegenelektrodeneinheit mit zwei Gegenelektroden gestatten, wobei die Messkreise in Zuordnung zu jeder der Gegenelektroden der Gegenelektrodeneinheit je einen zweiten Messkreis umfassen, in welchem nur die betreffende Gegenelektrode der Gegenelektrodeneinheit liegt, die jeweils andere Gegenelektrode der Gegenelektrodeneinheit jedoch nicht. Bei dieser Variante können die Messkreise ferner einen ersten Messkreis umfassen, in dem die Gegenelektroden der Gegenelektrodeneinheit elektrisch in Reihe liegen. Von den zweiten Messkreisen führt vorzugsweise mindestens einer, insbesondere jeder auch über die Anwendungselektrode.

Sofern die Schnittstellenanschlussanordnung den Anschluss wenigstens einer von der Anwendungselektrode gesonderten Nadelelektrode gestattet, kann mindestens einer, insbesondere jeder der zweiten Messkreise auch über die Nadelelektrode, jedoch nicht über die Anwendungselektrode führen. Bei der Nadelelektrode handelt es sich um eine Elektrode, welche das Hautgewebe durchstechen kann und in der Regel unter der Haut angebracht wird. Statt einer Nadelelektrode besteht auch die Möglichkeit, eine außen auf die Haut aufzubringende Klebeelektrode zu verwenden.

Die Messkreise können einen über die Anwendungselektrode und mehrere, insbesondere alle Gegenelektroden führenden vierten Messkreis umfassen, in dem die Gegenelektroden elektrisch parallel zueinander liegen.

Die Impedanzmessmittel können dazu ausgebildet sein, elektrische Hilfssignale unterschiedlicher, von der Frequenz der Behandlungsenergie jeweils verschiedener Frequenzen zu erzeugen und jedes der Hilfssignale in je mindestens einen Messkreis für die Impedanzmessung in diesem Messkreis einzuspeisen. Dabei können Filtermittel vorgesehen sein, welche für zumindest einen Teil der Hilfssignalfrequenzen sperrend wirken, jedoch für die Frequenz der Behandlungsenergie und ggf. mindestens eine andere Hilfssignalfrequenz durchlässig sind. Solche Filtermittel können insbesondere in Schaltungszweigen liegen, über welche der vom Generator abgegebene Behandlungsstrom fließt. Sie können dazu dienen sicherzustellen, dass bestimmte Hilfssignale nur in bestimmten Schaltungsteilen fließen, um die Impedanzmessung nicht zu verfälschen.

Alternativ oder zusätzlich zu einer Messung der Impedanzen in verschiedenen Messkreisen in Frequenzmultiplextechnik kann eine Zeitmultiplextechnik eingesetzt werden, bei der die Impedanzmessung in verschiedenen Messkreisen in unterschiedlichen Zeitschlitzen durchgeführt wird.

Die Vorrichtung kann eine Auswerte- und Steueranordnung enthalten, welche dazu ausgebildet ist, mindestens eine für die Impedanz in einem der Messkreise repräsentative Impedanzmessgröße und/oder mindestens eine aus den Impedanzmessgrößen eines oder mehrerer Messkreise abgeleitete Größe zu überwachen und abhängig davon eine vorbestimmte Reaktion zu bewirken, dass wenigstens eine Impedanzmessgröße und/oder wenigstens eine abgeleitete Größe eine vorbestimmte Bedingung erfüllen.

Dabei kann die Auswerte- und Steueranordnung die vorbestimmte Reaktion insbesondere abhängig davon bewirken, dass eine Impedanzmessgröße und/oder eine abgeleitete Größe einen abhängig von wenigstens einem vorherigen Wert der Impedanzmessgröße beziehungsweise der abgeleiteten Größe festgelegten Schwellenwert erreicht. Eine solche dynamische Festlegung von Schwellenwerten kann den Besonderheiten des jeweiligen Patienten und der jeweils verwendeten Apparatur Rechnung tragen.

Gemäß einer Weiterbildung der vorliegenden Erfindung kann die Auswerte- und Steueranordnung dazu ausgebildet sein, den Schwellenwert abhängig von einem gemessenen Extremum der Impedanzmessgröße beziehungsweise der abgeleiteten Größe festzulegen. Das Extremum kann beispielsweise der während einer Behandlung minimal gemessene Impedanzwert in einem der Messkreise sein. Der Schwellenwert kann dann beispielsweise durch Erhöhung des gemessenen Minimums um einen absoluten oder prozentualen Betrag festgelegt werden.

Die Auswerte- und Steueranordnung kann ferner dazu ausgebildet sein, das Verhältnis der Impedanzmessgrößen zweier verschiedener Messkreise als eine abgeleitete Größe zu ermitteln. Solche Verhältnisgrößen können hilfreich für die Erkennung der konkreten Gegenelektrode sein, bei der der Kontakt schlechter wird und deshalb Hautverbrennungen drohen. Dies kann insbesondere dadurch gelingen, dass die Impedanzmessgrößen zweier verschiedener Messkreise jeweils ins Verhältnis zur Impedanzmessgröße eines weiteren Messkreises gesetzt werden und der zeitlichen Verlauf der so gebildeten abgeleiteten Größen im gegenseitigen Vergleich überwacht wird.

Die vorbestimmte Reaktion kann eine Veränderung der Energieabgabe des Generators umfassen. Zusätzlich oder alternativ kann die vorbestimmte Reaktion auch die Ausgabe eines Warnsignals (beispielsweise in Form einer Meldung auf einer Anzeige oder in Form eines Wamtons) umfassen.

Die Erfindung wird nachfolgend anhand der beigefügten Zeichnungen weiter erläutert Die in den Figuren 5-7 gezeigten Aus führungs-beispiele sind nicht Teil den Erfindung. Es stellen dar
- Figur 1: eine schematische Blockdarstellung eines ersten Ausführungsbeispiels eines Chirurgiegeräts für die hochfrequente thermochirurgische Behandlung von Körpergewebe,
- Figur 2: einen Schaltplan einer Impedanzmesszelle des Chirurgiegeräts der Fig. 1;
- Figur 3: die Zusammenschaltung mehrerer Impedanzmesszellen des Chirurgiegeräts der Figur 1;
- Figur 4: eine schematische Blockdarstellung eines zweiten Ausführungsbeispiels eines HF-Chirurgiegeräts,
- Figur 5: eine schematische Blockdarstellung eines dritten Ausführungsbeispiels eines HF-Chirurgiegeräts,
- Figur 6: eine schematische Blockdarstellung eines vierten Ausführungsbeispiels eines HF-Chirurgiegeräts, und
- Figur 7: eine schematische Blockdarstellung eines fünften Ausführungsbeispiels eines HF-Chirurgiegeräts.

Gleiche oder gleichwirkende Komponenten sind in den Figuren durch gleiche Bezugszeichen bezeichnet.

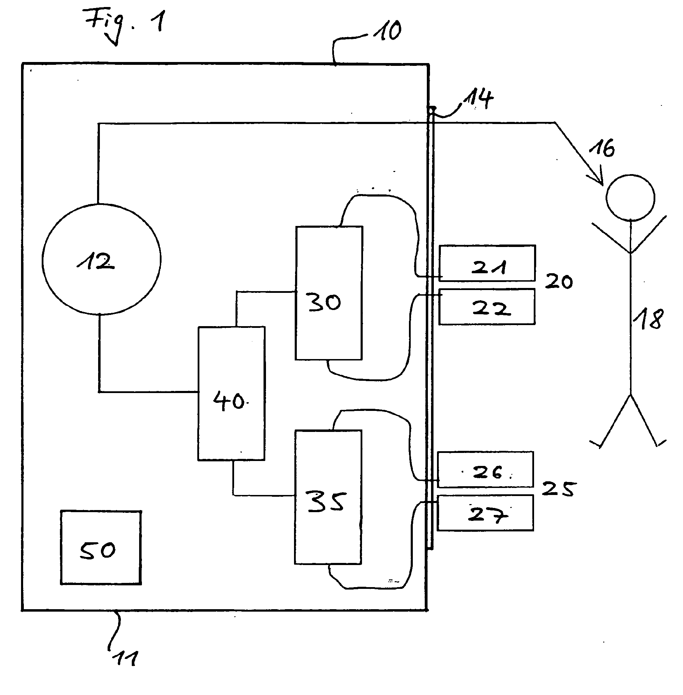

Das HF-Chirurgiegerät nach dem Ausführungsbeispiel der Fig. 1 ist allgemein mit 10 bezeichnet. Es besitzt ein Gerätegehäuse 11, in dem verschiedene elektrische oder/und elektronische Komponenten untergebracht sind. Die in dem Gerätegehäuse 11 untergebrachten Komponenten umfassen einen Wechselstromgenerator 12, welcher elektrische Behandlungsenergie in Form einer hochfrequenten Wechselspannung bereitstellt, deren Frequenz beispielsweise im mittleren dreistelligen kHz-Bereich liegt (z.B. 300 oder 500 kHz). Ferner umfassen diese Komponenten mehrere (im Beispielfall drei) Impedanzmesszellen 30, 35, 40 sowie eine in nicht näher dargestellter Weise mit dem Generator 12 und den Impedanzmesszellen verbundene elektronische Auswerte- und Steuerschaltung 50. An der Außenseite des Gehäuses 11 sind mehrere z.B. als Steckbuchsen ausgebildete Anschlussstellen vorgesehen, an denen in an sich bekannter Weise Elektrodenkabel an das Chirurgiegerät anschließbar sind. Die Anschlussstellen bilden zusammen eine mit 14 bezeichnete Schnittstellenanschlussanordnung des Chirurgiegeräts 10. Die Elektrodenkabel tragen an ihren distalen Enden eine oder mehrere Elektroden, die am oder im Körper eines zu behandelnden Patienten 18 anzubringen sind.

Speziell gestattet die Schnittstellenanschlussanordnung 14 bei dem gezeigten Ausführungsbeispiel den Anschluss mindestens einer Anwendungselektrode 16 und mindestens zweier Gegenelektrodeneinheiten 20, 25. Die Anwendungselektrode 16 wird in unmittelbarer Nähe der zu denaturierenden oder anderweitig thermisch zu behandelnden Stelle des Körpers positioniert. In der Regel wird sie in den Körper des Patienten 18 eingeführt, etwa um sie an der Herzwand zu positionieren und dort eine Erregungsbahn (Leitungsbahn) zu durchtrennen. Die Gegenelektrodeneinheiten 20, 25 weisen jeweils zwei flächige Gegenelektroden 21, 22 bzw. 26, 27 auf, die für eine monopolare Behandlungstechnik außen auf die Haut des Patienten 18 aufgelegt werden, so dass sie möglichst ganzflächigen Kontakt mit der Haut haben. Beispielsweise werden die Gegenelektrodeneinheiten 20, 25 auf den Rücken des Patienten, einen Oberschenkel oder die Brust aufgelegt, wobei zur Verbesserung des Kontakts oftmals ein elektrisch leitfähiges Gel auf die hautzugewandte Elektrodenunterseite aufgetragen wird. Die Gegenelektrodeneinheiten 20, 25 weisen in nicht näher dargestellter Weise jeweils ein Trägersubstrat auf, das als gemeinsamer Träger für die beiden Elektroden der betreffenden Gegenelektrodeneinheit dient. Solche Gegenelektrodeneinheiten mit zwei an einem gemeinsamen Trägersubstrat angeordneten, elektrisch jedoch unverbundenen Flächenelektroden sind in der Fachwelt auch als geteilte Gegenelektroden bekannt.

Statt Gegenelektrodeneinheiten mit jeweils zwei (oder sogar mehr) körperlich zusammenhängenden Flächenelektroden zu verwenden, ist es prinzipiell vorstellbar, Einzelelektroden für wenigstens einen Teil der Gegenelektroden 21, 22, 26, 27 zu verwenden.

Der vom Generator 12 abgegebene elektrische Behandlungsstrom fließt über die Anwendungselektrode 16 in das Körpergewebe des Patienten 18. Der Stromrückfluss findet über die Gegenelektroden 21, 22, 26, 27 statt. Wegen der erheblich größeren Fläche jeder der Gegenelektroden 21, 22, 26, 27 im Vergleich zur Anwendungselektrode 16 ist die Stromdichte an den Gegenelektroden 21, 22, 26, 27 erheblich kleiner als an der Anwendungselektrode 16, vorausgesetzt ein großflächiger, guter Kontakt der Gegenelektroden 21, 22, 26, 27 mit der Haut des Patienten 18 bleibt gewahrt. Sobald sich jedoch die wirksame Kontaktfläche einer Gegenelektrode mit der Haut verringert, etwa weil sich die Gegenelektrode teilweise von der Haut ablöst oder weil unter einem Teil der Gegenelektrode starke Schweißbildung auftritt, die lokal die Leitfähigkeit stark herabsetzen kann, kann es zu örtlichen Spitzen der Stromdichte an der Gegenelektrode kommen. Solche Stromdichtespitzen bringen die Gefahr einer Verkohlung der Haut mit sich.

Zweck der Impedanzmesszellen 30, 35, 40 ist es, durch Impedanzmessungen unerwünschte Hautverkohlungen an den Gegenelektroden rechtzeitig zu erkennen und zu vermeiden.

Jede der Impedanzmesszellen 30, 35, 40 dient zur messtechnischen Erfassung der Impedanz in einem Impedanzmesskreis. Wegen der Mehrzahl der vorhandenen Impedanzmesszellen 30, 35, 40 ist somit eine Impedanzmessung in mehreren Impedanzmesskreisen möglich. Jeder dieser Impedanzmesskreise führt über eine oder mehrere der an die Schnittstellenanschlussanordnung 14 anschließbaren Elektroden. Die Impedanzmesszellen 30, 35, 40 liefern ihre erfassten Impedanzmessgrößen an die Auswerte- und Steuerschaltung 50, welche die erhaltenen Impedanzmessgrößen auswertet und die Energieabgabe des Generators 12 abhängig von den gemessenen Impedanzen steuert.

Im konkreten Beispielfall der Fig. 1 erfasst die Impedanzmesszelle 30 die Impedanz in einem Messkreis, welcher von der Messzelle 30 über die Gegenelektrode 21 und den Körper der Patienten bis zur Gegenelektrode 22 und von dort wieder zur Messzelle 30 führt. Die beiden Gegenelektroden 21 und 22 liegen in diesem Messkreis demnach elektrisch in Reihe. Der für die Impedanz in dem Messkreis wirksame Teil des Patientenkörpers beschränkt sich im wesentlichen auf das Hautstück und das darunterliegende Gewebe zwischen den beiden Gegenelektroden 21, 22. Wegen der körperlich zusammenhängenden Ausbildung der beiden Gegenelektroden 21, 22 als Gegenelektrodeneinheit ("geteilte Gegenelektrode") liegt nur ein relativ kurzes Hautstück elektrisch zwischen den beiden Gegenelektroden 21, 22.

Die Impedanzmesszelle 35 erfasst dagegen die Impedanz in einem Messkreis, welcher von der Messzelle 35 über die Gegenelektrode 26 und den Körper des Patienten bis zur Gegenelektrode 27 und von dort wieder zur Messzelle 35 führt. Auch hier liegen die beiden Gegenelektroden 21 elektrisch in Reihe in dem Messkreis.

In der in den Ansprüchen verwendeten Terminologie repräsentieren die beiden Messkreise, in denen die Impedanzmesszellen 30, 35 die Impedanz messen, jeweils einen ersten Messkreis. Denn in ihnen liegen die Gegenelektroden je einer Gegenelektrodeneinheit elektrisch in Reihe miteinander.

Die Impedanzmesszelle 40 erfasst die Impedanz in einem Messkreis, welcher von der Messzelle 40 über die Gegenelektrodeneinheit 20, von dort über den Patientenkörper zur Gegenelektrodeneinheit 25 und zurück zur Messzelle 40 führt. In diesem Messkreis liegen somit die beiden Gegenelektrodeneinheiten 20, 25 elektrisch in Reihe in miteinander, wobei die einzelnen Gegenelektroden jeder der beiden Gegenelektrodeneinheiten jeweils elektrisch parallel zueinander liegen (d.h. die Elektroden 21, 22 liegen parallel zueinander und die Elektroden 26, 27 liegen parallel zueinander). Der für die Messkreisimpedanz wirksame Teil des Patientenkörpers ist in diesem Fall größer als in den Fällen der Messkreise der Messzellen 30, 35, da der Abstand der beiden Gegenelektrodeneinheiten 20, 25 voneinander regelmäßig größer sein wird als der gegenseitige Abstand der beiden Elektroden einer Gegenelektrodeneinheit.

In der Terminologie der Ansprüche stellt der von der Messzelle 40 vermessene Messkreis einen dritten Messkreis dar.

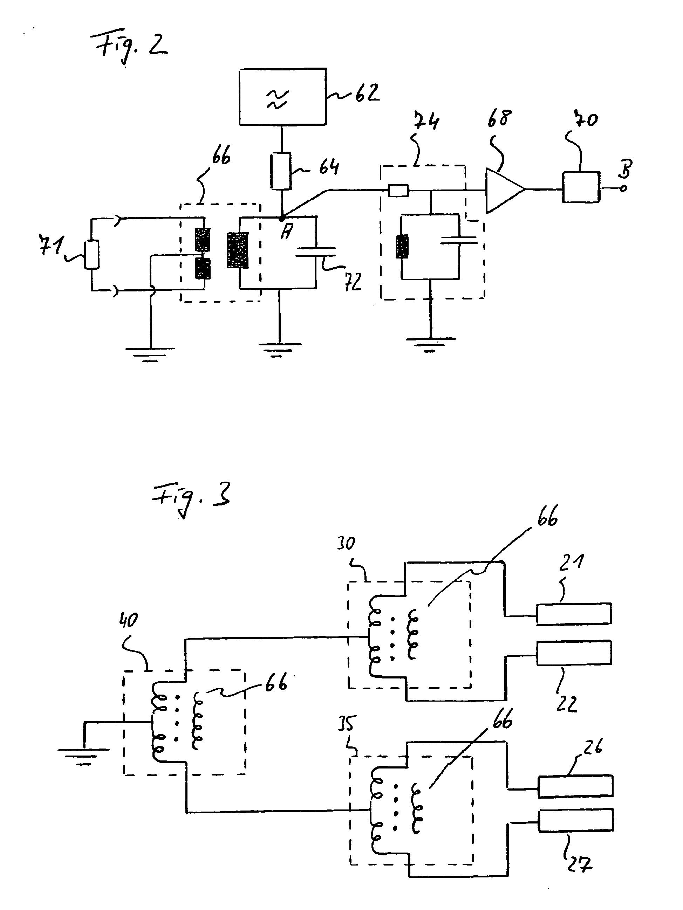

Das Schaltbild der Fig. 2 zeigt einen beispielhaften Aufbau einer Impedanzmesszelle, die für die Impedanzmessung zwischen Gegenelektroden oder zwischen Gegenelektrodeneinheiten verwendet werden kann. Die Messzelle arbeitet mit einem elektrischen Hilfssignal in Form einer von einem Wechselspannungsgenerator 62 bereitgestellten Wechselspannung, deren Frequenz von der Frequenz des Behandlungsstroms des Generators 12 verschieden ist. Da das Hilfssignal keine physiologische Wirkung auf den Körper entfalten soll, liegt seine Frequenz beispielsweise bei etwa 100 kHz, jedenfalls aber deutlich unter der Behandlungsfrequenz. In Reihe mit dem Generator 62 liegt ein Widerstand 64, dessen Widerstandswert groß im Vergleich zu den restlichen Impedanzen im Impedanzmesskreis ist, insbesondere groß im Vergleich zur Gewebeimpedanz des Körpers 18 und zu den Übergangswiderständen zwischen Haut und Gegenelektroden. Der Generator 62 bildet zusammen mit dem Widerstand 64 eine Wechselstromquelle konstanter Wechselstromamplitude.

Der von dem Generator 62 und dem Widerstand 64 erzeugte Hilfswechselstrom wird in die Primärseite eines Übertragers 66 eingespeist. Sekundärseitig wird so eine Wechselspannung gleicher Frequenz induziert. Die Sekundärseite des Übertragers 66 ist über zwei oder mehr der an das Chirurgiegerät 10 angeschlossenen Elektroden in einem geschlossenen Stromkreis elektrisch mit dem Körper 18 des Patienten verbunden, so dass über die Elektroden ein Sekundärstrom zwischen der Sekundärseite des Übertragers 66 und dem Körper 18 des Patienten fließt. Je nach der zwischen den Elektroden wirksamen Gewebe- bzw. Hautimpedanz und dem Kontaktwiderstand zwischen den Elektroden und der Haut (gemeinsam dargestellt durch einen Lastwiderstand 71) wird dann der Sekundärkreis unterschiedlich belastet, was sich in einer entsprechend unterschiedlichen Belastung (Dämpfung) der Primärseite des Übertragers 66 niederschlägt. Die Spannungsamplitude auf der Primärseite an einem Punkt A hängt von der Dämpfung der Primärseite und folglich von der Hautimpedanz und der Kontaktimpedanz zwischen Haut und angelegten Elektroden ab. Es hat sich gezeigt, dass diese Abhängigkeit in weiten Bereichen zumindest näherungsweise proportional ist.

Die Spannung am Punkt A wird abgegriffen, mittels einer Verstärkeranordnung 68 verstärkt und anschließend mittels einer Gleichrichterschaltung 70 gleichgerichtet. Am Ausgang B der Gleichrichterschaltung 70 steht somit eine Gleichspannung bereit, deren Höhe ein Maß für die Gesamtimpedanz im Sekundärkreis ist. Verändert sich die Höhe dieser Gleichspannung, deutet dies auf eine beispielsweise durch beginnende Verkohlung bedingte Veränderung der Gewebeimpedanz oder/und auf einen veränderten Kontaktwiderstand aufgrund teilweiser Ablösung einer oder mehrerer Elektroden hin. Die Gleichspannung am Punkt B, die eine Impedanzmessgröße im Sinne der Erfindung darstellt, wird von der Auswerte- und Steuerschaltung 50 des Chirurgiegeräts 10 überwacht und ausgewertet. Ggf. kann sie hierzu in einen digitalen Wert umgewandelt werden.

Man erkennt in Fig. 2 ferner, dass die Sekundärseite des Übertragers 66 eine Mittenanzapfung besitzt, von der aus der Behandlungsstrom direkt oder über mindestens einen weiteren Übertrager 66 nach Masse fließt. Der über die Anwendungselektrode 16 in den Körper 18 eingespeiste Behandlungsstrom fließt über die mit der Sekundärseite des Übertragers 66 verbundenen Gegenelektroden gegenphasig in die beiden durch die Mittenanzapfung getrennten Sekundärspulen. Über die Mittenanzapfung wird der HF-Stromkreis dann geschlossen. Wegen der Gegenphasigkeit der in den Sekundärspulen fließenden Ströme heben sich die zugeordneten Magnetfelder auf. Auf der Primärseite des Übertragers 66 wird daher keine Spannung infolge des Behandlungsstroms induziert. Der Übertrager 66 verhält sich sozusagen passiv bei der Behandlungsfrequenz. Dies gilt zumindest solange, wie die Behandlungsteilströme in den beiden Sekundärspulen gleich groß sind. Bei Ungleichheit der Behandlungsteilströme in den Sekundärspulen des Übertragers 66 wird die Differenz zur Primärseite transformiert. Das heißt, neben der Hilfssignalfrequenz können auf der Primärseite unter Umständen auch andere Frequenzen auftreten, wie z.B. die Behandlungsfrequenz im Fall unsymmetrischer Behandlungsteilströme in den Sekundärspulen. Solche anderen Frequenzen sollen jedoch möglichst keinen Einfluss auf die Impedanzmessgröße am Punkt B haben, denn mit der vorliegenden Schaltung wird eine Impedanzmessung angestrebt, die allein die Reaktion (Antwort) auf die Anregung durch den Hilfsstrom misst.

Um die Impedanzmessgröße am Punkt B von unerwünschten Störeinflüssen zu befreien, enthält die Schaltung gemäß Fig. 2 geeignete Filtermittel, welche als Bandpass für die Hilfssignalfrequenz wirken und als Sperre für andere unter Umständen auftretende Frequenzen, insbesondere die Behandlungsfrequenz, wirken. Diese Filtermittel bilden im gezeigten Beispielfall zwei Filterstufen, deren erste von einem LC-Parallelkreis gebildet ist, der sich aus der Primärspule des Übertragers 66 und einem parallel hierzu geschalteten Kondensator 72 zusammensetzt. Im Anschluss daran ist eine weitere Filterstufe in Form eines RLC-Bandpassfilters 74 vorgesehen. Die beiden Filterstufen gewährleisten, dass am Eingang der Verstärkeranordnung 68 im wesentlichen nur Signale mit der Hilfssignalfrequenz des Generators 62 auftreten.

Die verschiedenen Impedanzmesszellen 30, 35, 40 des Chirurgiegeräts 10 der Fig. 1 sind alle gleich aufgebaut. Jede enthält ihren eigenen Übertrager 66, ihre eigenen Filterkomponenten 72, 74, ihre eigene Verstärkeranordnung 68 und ihre eigene Gleichrichteranordnung 70. Der Hilfssignalgenerator 62 (mit oder ohne den Widerstand 64) kann für alle Impedanzmesszellen 30, 35, 40 gemeinsam vorgesehen sein.

Fig. 3 zeigt, wie die in den Messzellen 30, 35, 40 enthaltenen Übertrager 66 miteinander und mit den Gegenelektroden 21, 22, 26, 27 zusammengeschaltet sind. Die Impedanzmesszellen 30, 35 sind unmittelbar mit den Gegenelektroden 21, 22 bzw. 26, 27 verbunden. Hierzu sind die betreffenden Gegenelektroden mit je einem der beiden äußeren sekundären Übertrageranschlüsse der betreffenden Messzelle verbunden. Die Impedanzmesszelle 40 dagegen ist nicht unmittelbar mit den Gegenelektroden 21, 22, 26, 27 verbunden. Bei ihr sind die äußeren sekundären Übertrageranschlüsse jeweils mit dem mittleren sekundären Übertrageranschluss (d.h. der Mittenanzapfung) der Messzelle 30 bzw. der Messzelle 35 verbunden, während die sekundäre Mittenanzapfung der Messzelle 40 mit Masse verbunden ist und so den Rückflussweg für den Behandlungsstrom schließt.

Um ein zahlenmäßiges Beispiel zu geben, kann an den Widerstand 64 eine Hilfswechselspannung von etwa 1 V angelegt werden. Bei einem Widerstandswert des Widerstands 64 von etwa 100 kOhm ergibt sich dadurch ein Messstrom von etwa 10 µA abzüglich etwaiger Schaltungsverluste. Im bedämpften Zustand (bei angelegten Elektroden) stehen auf der Primärseite des Übertragers 66 dann typischerweise Wechselspannungen in der Größenordnung von 1 mV bei der Frequenz der Hilfswechselspannung (z.B. 100kHz). Im Vergleich hierzu kann über die Anwendungselektrode 16 ein Behandlungsstrom mit bis zu etwa 3 A bei einer Spannung von etwa 200 V abgegeben werden. Dieser Strom fließt im Idealfall je hälftig durch beide Kontaktflächen des Ersatzwiderstands 71 zurück zur Masse. Wegen der Gegenphasigkeit der Teilströme in den beiden Sekundärwicklungen des Übertragers 66 heben sich die Magnetfelder dann auf. Im Idealfall wird also keine HF-Spannung von der Sekundärseite zur Primärseite übertragen. Sobald die Ströme aber asymmetrisch durch die beiden Sekundärwicklungen laufen, wird die Differenz in die Primärwicklung transformiert. Es kann sich dann am Punkt A eine HF-Spannung von bis zu etwa 100 V bei der Behandlungsfrequenz (z.B. 500 kHz) einstellen. Gegenüber dieser Störspannung verschwindet nahezu die Impedanzmessspannung zur Ermittlung des Hautwiderstands (ca. 1 mA, siehe oben). Der Kondensator 72 und insbesondere das nachgeschaltete Bandpassfilter 74 filtern dann die "zarte" Impedanzmessspannung heraus.

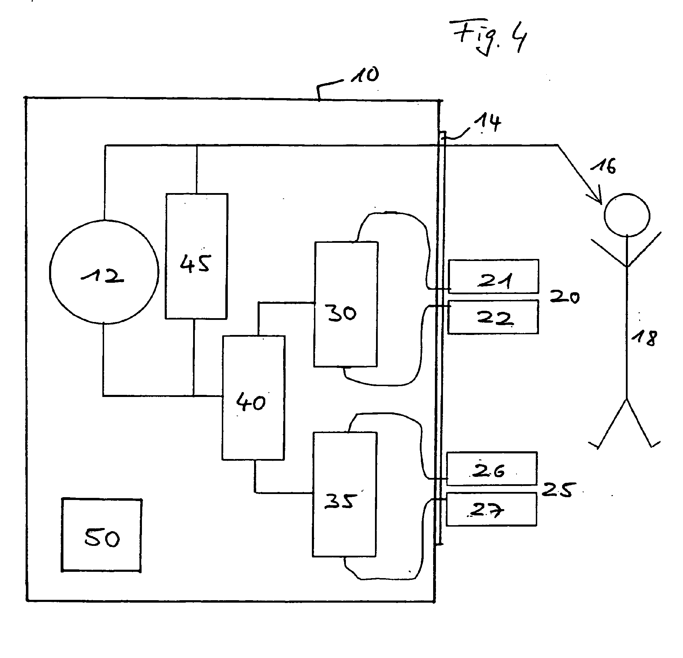

Bevor auf Einzelheiten der Auswertung der gemessenen Impedanzen und der resultierenden Steueraktionen durch die Auswerte- und Steuerschaltung 50 eingegangen wird, seien zuvor noch anhand der Figuren 4 bis 7 weitere beispielhafte Konfigurationen von Impedanzmesskreisen schematisch dargestellt.

Die Konfiguration nach Figur 4 enthält neben den Impedanzmesszellen 30, 35, 40 einen Impedanzmessblock 45, welcher der Impedanzmessung in einem Messkreis dient, der über die Anwendungselektrode 16, den Körper 18 des Patienten und alle Gegenelektroden 21, 22, 26, 27 führt. Man erkennt, dass in diesem Messkreis die Gegenelektrodeneinheiten 20, 25 zueinander elektrisch parallel und in Reihe mit der Anwendungselektrode liegen. Im Sinne der in den Ansprüchen verwendeten Terminologie handelt es sich um einen vierten Impedanzmesskreis. Der Impedanzmessblock 45 kann die Impedanz in seinem Messkreis beispielsweise aus dem Verhältnis zwischen Spannungsamplitude und Stromamplitude (und ggf. der relativen Phasenlage zwischen Spannung und Strom) eines zusammen mit dem Behandlungsstrom über die Anwendungselektrode 16 in den Körper eingespeisten Hilfssignals ermitteln. Entsprechende Techniken zur Impedanzermittlung sind in der Fachwelt an sich bekannt.

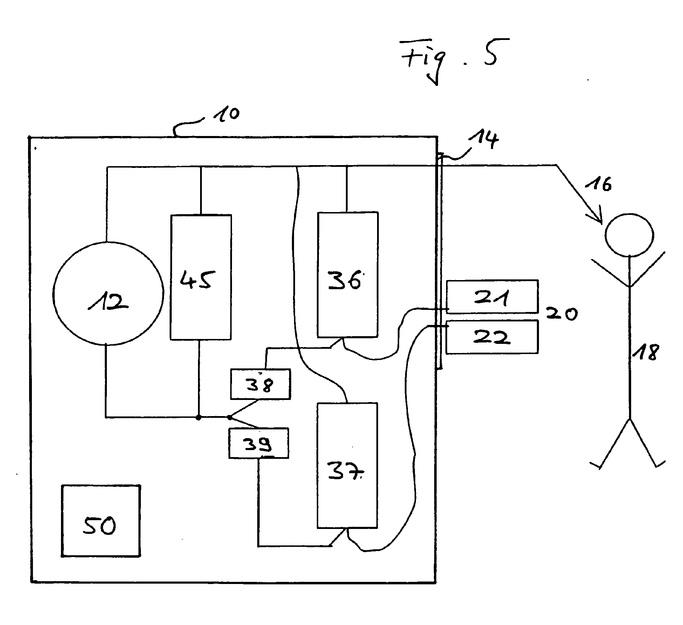

Das Chirurgiegerät nach Figur 5 enthält Impedanzmessblöcke 36, 37 und 45. Der Impedanzmessblock 45 dient wie in Fig. 4 der Impedanzmessung in einem über die Anwendungselektrode 16, den Patientenkörper 18 und alle angeschlossenen Gegenelektroden führenden Messkreis. Im gezeigten Beispielfall ist nur die Gegenelektrodeneinheit 20 im Einsatz; deswegen liegen in dem von dem Block 45 vermessenen Messkreis nur die beiden Gegenelektroden 21, 22 (nämlich in Reihe mit der Anwendungselektrode 16 und parallel zueinander). Die beiden Messblöcke 36, 37 gestatten dagegen jeweils die Impedanzmessung in einem Messkreis, welcher über die Anwendungselektrode 16, den Körper 18 und nur je eine der beiden Gegenelektroden 21, 22 führt. Konkret liegt im gezeigten Beispielfall die Gegenelektrode 21 in dem von dem Messblock 36 vermessenen Messkreis, während die Gegenelektrode 22 in dem von dem Messblock 37 vermessenen Messkreis liegt. Bei den durch die Messblöcke 36, 37 vermessenen Messkreisen handelt es sich im Sinne der Anspruchsterminologie jeweils um einen zweiten Messkreis.

Diese beiden zweiten Messkreise werden ähnlich wie der vom Messblock 45 vermessene Messkreis mit einem Hilfssignal aus einer nicht näher dargestellten Hilfssignalquelle gespeist. Auch hier haben die Hilfssignale wieder eine von der Behandlungsfrequenz verschiedene Frequenz. Außerdem unterscheiden sich die Hilfssignalfrequenzen der verschiedenen Messkreise, um gleichzeitig in allen Messkreisen Impedanzmessungen durchführen zu können.

Es ist nun folgendes Problem zu lösen. Der Generator 12 liefert Strom über die Anwendungselektrode 16 an den Patienten 18, wobei dieser Behandlungsstrom über die Gegenelektroden 21, 22 zurück zum Generator 12 bzw. zur Masse fließt. Würden die beiden Gegenelektroden 21, 22 jedoch ohne weitere Vorkehrungen einfach zusammengeschaltet, um den Rückfluss des Behandlungsstroms von den beiden Gegenelektroden 21, 22 zu gewährleisten, entstünde ein Kurzschluss zwischen den beiden Rückflussleitungen, die von den Elektroden 21, 22 in Richtung zum Generator 12 ausgehen. In diesem Fall würden die Impedanzmessblöcke 36, 37 gleiche Messergebnisse liefern, da in beiden Messkreisen jeweils beide Gegenelektroden 21, 22 parallel lägen. Abhilfe wird mittels einer hier aus zwei Bandsperrfiltern 38, 39 bestehenden Filteranordnung geschaffen, welche durchlässig für die Behandlungsfrequenz und die Messsignalfrequenz des Impedanzmessers 45 ist. Außerdem wirkt die Filteranordnung 38, 39 sperrend für die Messsignalfrequenzen der Messblöcke 36, 37. Auf diese Weise wird verhindert, dass das über die Anwendungselektrode 16 in den Körper 18 des Patienten eingespeiste Messsignal des Messblocks 36 nicht alleine über die Gegenelektrode 21 zum Messblock 36 zurückfließt sondern auch über die Gegenelektrode 22. Das heißt, die Filteranordnung 38, 39 stellt sicher, dass der Messblock 36 die Impedanz in einem Messkreis misst, in dem allein die Gegenelektrode 21 liegt, nicht jedoch die Gegenelektrode 22. Gleichermaßen verhindert die Filteranordnung 38, 39, dass das Messsignal des Messblocks 37 über die Gegenelektrode 21 zum Messblock 37 zurückfließen kann und so dessen Impedanzmessung verfälscht.

Um ein Beispiel in Zahlen zu geben, kann der Messblock 36 eine Messsignalfrequenz von 100 kHz und der Messblock 37 eine Messsignalfrequenz von 105 kHz verwenden, während der Messblock 45 beispielsweise eine Messsignalfrequenz von 50 kHz verwenden kann. Das Filter 38 kann dabei passend zum Messblock 36 auf 100 kHz Bandsperre ausgelegt sein und das Filter 39 passend zum Messblock 37 auf 105 kHz Bandsperre.

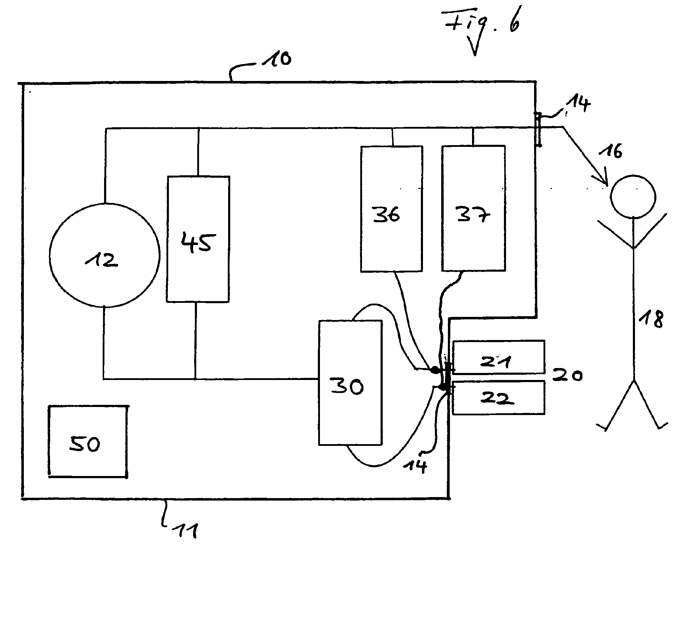

Figur 6 zeigt eine Variante mit einer Impedanzmesszelle 30 und Impedanzmessblöcken 36, 37 und 45. Der Übersichtlichkeit halber ist eine den Bandsperrfiltern 38, 39 der Fig. 5 entsprechende Filteranordnung in der Zeichnung weggelassen. Tatsächlich jedoch ist in Fig. 6 den beiden Messblöcken 36, 37 eine für die Behandlungsfrequenz und die Messsignalfrequenz des Messblocks 45 durchlässige Filteranordnung zugeordnet, welche für die Messsignalfrequenzen der beiden Messblöcke 36, 37 sperrend wirkt. Beispielsweise können geeignete Bandsperrfilter in den beiden Leitungszweigen angeordnet sein, welche die Gegenelektroden 21, 22 mit den äußeren Sekundäranschlüssen des in der Messzelle 30 enthaltenen Übertragers 66 (vgl. Fig. 2) verbinden. Zweckmäßigerweise unterscheidet sich die Messsignalfrequenz der Messzelle 30 von den Messsignalfrequenzen der beiden Messblöcke 36, 37 sowie von der Messsignalfrequenz des Messblocks 45 und der Behandlungsfrequenz.

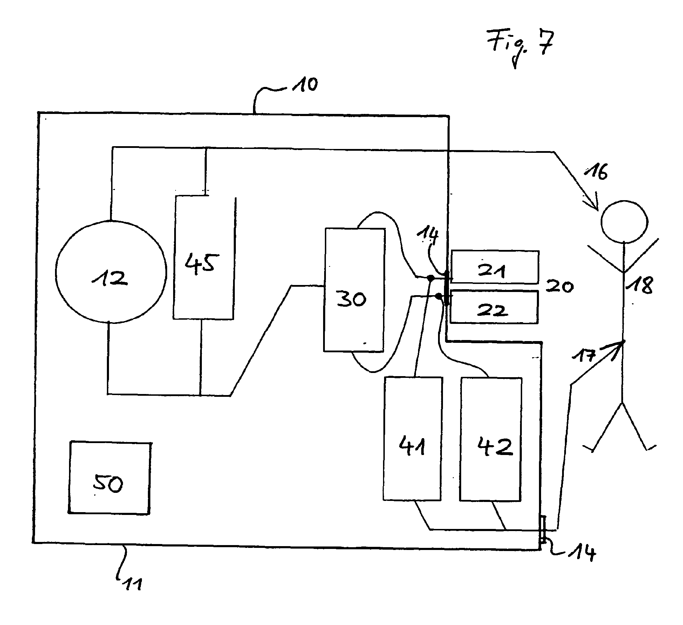

Figur 7 schließlich zeigt eine Variante, bei der zusätzlich zu einer Impedanzmesszelle 30 und einem Impedanzmessblock 45 zwei Impedanzmessblöcke 41, 42 vorgesehen sind, welche jeweils eine Impedanzmessung in einem über eine Nadelelektrode 17 führenden Impedanzmesskreis gestatten. Die Nadelelektrode 17 ist ebenfalls an der Schnittstellenanschlussanordnung 14 anschließbar und kann als Referenzelektrode verwendet werden, welche in einem Bereich zwischen der Anwendungselektrode 16 und den Gegenelektroden 21, 22 (im gezeigten Beispielfall befindet sich erneut nur die Gegenelektrodeneinheit 20 im Einsatz) am Körper 18 angebracht wird und dort ein Mittenpotential abgreift. Solche Nadelelektroden und ihre Verwendung sind in der Fachwelt an sich bekannt. Von Bedeutung im Zusammenhang mit dem Ausführungsbeispiel der Fig. 7 ist, dass die Nadelelektrode 17 für die Impedanzmessung genutzt wird. Konkret erfolgt mittels des Messblocks 41 eine Impedanzmessung in einem über die Gegenelektrode 21, den Patientenkörper 18 und die Nadelelektrode 17 führenden Messkreis. Mittels des Messblocks 42 ist dagegen eine Impedanzmessung in einem über die Gegenelektrode 22, den Patientenkörper 18 und die Nadelelektrode 17 führenden Messkreis ermöglicht. Die beiden über die Nadelelektrode 17 führenden Messkreise stellen weitere Beispiele jeweils eines zweiten Messkreises im Sinne der Anspruchsterminologie dar. Ähnlich wie bei den Konfigurationen nach Fig. 5 und 6 kann bei der Konfiguration der Fig. 7 den Messblöcken 41, 42 eine Filteranordnung (nicht näher dargestellt) zugeordnet sein, welche für die Messsignalfrequenzen der Messblöcke 41, 42 sperrend wirkt und für die übrigen vorkommenden Frequenzen (andere Messsignalfrequenzen, Behandlungsfrequenz) durchlässig ist. Eine solche Filteranordnung stellt ähnlich den vorigen Fällen sicher, dass der tatsächlich vermessene Messkreis des Messblocks 41 allein über die Gegenelektrode 21 führt und nicht auch über die Gegenelektrode 22 und dass der tatsächlich vermessene Messkreis des Messblocks 42 allein über die Gegenelektrode 22 führt.

Bei jeder der gezeigten Ausführungsformen erhält die Auswerte- und Steuerschaltung 50 somit mehrere für die gemessene Impedanz in je einem Messkreis repräsentative Impedanzmessgrößen. Die erhaltenen Impedanzmessgrößen nutzt die Auswerte- und Steuerschaltung 50 insbesondere zur Steuerung der Energieabgabe der Generators 12, um rechtzeitig reagieren zu können, wenn infolge der teilweisen Ablösung einer Gegenelektrode die Gefahr von Hautverkohlungen besteht. Wenigstens ein Teil der erhaltenen Impedanzmessgrößen kann von der Auswerte- und Steuerschaltung 50 unmittelbar überwacht und für die Steuerung des Chirurgiegeräts herangezogen werden. Zusätzlich kann die Auswerte- und Steuerschaltung 50 aus einer oder mehreren der Impedanzmessgrößen eine oder mehrere abgeleitete Größen ermitteln und diese bei der Steuerung des Chirurgiegeräts einzubeziehen. Abgeleitete Größen können beispielsweise eine Differenz zwischen zwei Impedanzmessgrößen und eine zeitliche Ableitung einer Impedanzmessgröße sein.

Insbesondere ermittelt die Auswerte- und Steuerschaltung 50 aus den gemessenen Impedanzgrößen mehrere (zwei oder mehr) Impedanzverhältnisse und vergleicht den zeitlichen Verlauf der ermittelten Impedanzverhältnisse. Vorzugsweise ist die Auswerte- und Steuerschaltung 50 dazu in der Lage, zwei in unterschiedlichen Impedanzmesskreisen erhaltene Impedanzmessgrößen jeweils ins Verhältnis zu einer in einem weiteren Impedanzmesskreis erhaltenen Impedanzmessgröße zu setzen und die so erhaltenen Verhältnisgrößen in ihrem zeitlichen Verlauf miteinander zu vergleichen. Auf diese Weise besteht die Möglichkeit festzustellen, welche der beiden Gegenelektroden einer Gegenelektrodeneinheit sich von der Haut abzulösen beginnt. Betrachtet man beispielsweise die Ausführungsform der Fig. 6, so können die von den beiden Messzellen 36, 37 gemessenen Impedanzen jeweils ins Verhältnis zu der von der Messzelle 45 gemessenen Impedanz gesetzt werden. Ändert sich eines dieser Verhältnisse, das andere jedoch nicht oder nicht im gleichen Maß, ist dies ein Indikator dafür, dass es ein Problem mit der Gegenelektrode gibt, deren ermitteltes Impedanzverhältnis sich ändert. In ähnlicher Weise ist es in Fig. 1 vorstellbar, die gemessenen Impedanzen der Messzellen 30, 35 jeweils ins Verhältnis zur gemessenen Impedanz der Messzelle 40 setzen. Es können aber selbstverständlich alternativ oder zusätzlich die gemessenen Impedanzen jeweils einzeln für sich ausgewertet und für die Steuerung des Generators 12 herangezogen werden.

Die Auswertung der erhaltenen Impedanzmessgrößen oder/und der hiervon abgeleiteten Größen kann eine statische Grenzwertkontrolle umfassen. Eine statische Grenzwertkontrolle kann darin bestehen, dass mindestens eine der Impedanzmessgrößen oder/und mindestens eine der hiervon abgeleiteten Größen mit mindestens einem vorgegebenen Schwellenwert verglichen wird, der bereits vor Beginn der Operation feststeht und sich während der Operation nicht ändert. Erreicht die betreffende Größe (Impedanzmessgröße oder abgeleitete Größe) den Schwellenwert, bewirkt die Auswerte- und Steuerschaltung 50 eine vorbestimmte Reaktion, indem sie beispielsweise die Energieabgabe des Generators 12 unterbricht oder absenkt oder erst gar nicht freigibt. Biologisches Gewebe ist jedoch kein statisches, reproduzierbares Gebilde. Nicht alle Patienten sind gleich. So können die tatsächlichen Gewebeimpedanzen von vielen Kriterien abhängen, etwa vom Alter des Patienten, vom Wassergehalt des Gewebes, von der örtlichen Lage der Elektroden am Körper, vom Fettgehalt, vom Salzgehalt, von der Durchblutung, von der Hautbeschaffenheit, von der Nähe zu den Knochen, von der Nähe zu Flüssigkeitsansammlungen und Gefäßen usw. Auch die Lagerung des Patienten und der Druck auf die Gegenelektroden können eine wichtige Rolle spielen. Ferner können sich die Größe, das Material und der gegenseitige Abstand der Gegenelektroden sowie die Eigenschaften eines zur Kontaktverbesserung verwendeten Gels beträchtlich auf das Impedanzverhalten auswirken. Allgemein gültige statische Schwellenwerte sind deshalb, wenn überhaupt, nur schwer festzulegen. Als sinnvoll hat sich die Festlegung statischer Schwellenwerte erwiesen, um gewisse maximale Grenzwerte nicht zu überschreiten. Dadurch können gewisse Mindestanforderungen eingehalten werden. Ausreichend ist eine solche statische Kontrolle regelmäßig jedoch nicht.

Deshalb umfasst die Messgrößenauswertung vorzugsweise auch eine dynamische Grenzwertkontrolle, bei der ein oder mehrere Schwellenwerte dynamisch erst im Verlauf der Operation festgelegt werden. Insbesondere wird ein solcher Schwellenwert abhängig vom gemessenen Wert mindestens einer der Impedanzmessgrößen oder/und mindestens einer abgeleiteten Größe festgelegt und ggf. angepasst, sollte sich der gemessene Wert der Impedanzmessgröße bzw. der abgeleiteten Größe während der Operation verändern. Dem liegt die Erkenntnis zugrunde, dass die in einem Impedanzmesskreis gemessene Impedanz eine starke Abhängigkeit vom Druck haben kann, mit dem eine Gegenelektrode gegen die Haut des Patienten gedrückt wird. Druckänderungen können dann schon zu signifikanten Änderungen der gemessenen Impedanz führen, ohne dass dabei eine Teilablösung der Gegenelektrode von der Haut stattfinden würde.

Bei der dynamischen Schwellenwertfestlegung wird für eine fortlaufend (d.h. kontinuierlich oder in Intervallen) gemessene Impedanzmessgröße oder eine abgeleitete Größe ein Extremwert (Minimum oder Maximum) ermittelt und gespeichert. Wird der gespeicherte Extremwert im weiteren Verlauf der Messung von der Impedanzgröße bzw. der abgeleiteten Größe unterschritten (im Fall eines Minimums) oder überschritten (im Fall eines Maximums), erfolgt eine Aktualisierung des gespeicherten Extremwerts. Diese Extremwertermittlung kann über die gesamte Dauer der thermochirurgischen Behandlung durchgeführt werden. Es ist auch vorstellbar, die Extremwertermittlung auf einem bestimmten Teil der Behandlungsdauer zu beschränken, etwa auf eine Anfangsphase der Behandlung. Abhängig von dem gespeicherten Extremwert wird nun mindestens ein Schwellenwert festgelegt, beispielsweise indem der gespeicherte Extremwert um einen vorbestimmten, gewünschtenfalls vom Benutzer vorgebbaren prozentualen oder absoluten Anteil erhöht oder erniedrigt wird. Auf diese Weise kann ein für den jeweiligen Patienten und das jeweils verwendete Gerätesystem individuell geeigneter Schwellenwert festgelegt werden. Eine Änderung des gespeicherten Extremwerts geht dann mit einer Änderung des abhängig hiervon ermittelten Schwellenwerts einher. Wird der in dieser Weise dynamisch festgelegte Schwellenwert im Verlauf der Operation erreicht, bewirkt die Auswerte- und Steuerschaltung 50 ähnlich wie bei der statischen Grenzwertkontrolle eine vorbestimmte Reaktion. Diese kann insbesondere eine Veränderung der Energieabgabe des Generators 12 umfassen. Alternativ oder zusätzlich ist es vorstellbar, dass bei Erreichen eines dynamisch festgelegten Schwellenwerts oder/und eines statisch festgelegten Schwellenwerts ein Warnsignal (optisch, akustisch) ausgegeben wird, das dem Benutzer auf den kritischen Kontaktwiderstand der Gegenelektroden hinweist.

Das obige Prinzip der dynamischen Grenzwertkontrolle sei an einem Zahlenbeispiel illustriert. Betrachtet sei dabei die Impedanzmessung über die Reihenschaltung der Gegenelektroden 21, 22 mittels der Messzelle 30 in Fig. 1 (entsprechendes gilt für die Impedanzmessung über die Reihenschaltung der Gegenelektroden 26, 27 mittels der Messzelle 35). Sobald die Gegenelektrodeneinheit 20 mit den beiden Gegenelektroden 21, 22 am Körper 18 des Patienten angebracht wird, ergibt sich aus Sicht des Chirurgiegeräts 10 eine Impedanzänderung von unendlich (kein Kontakt der Gegenelektrodeneinheit 20 mit dem Patienten) auf einen endlichen, niedrigeren Impedanzwert. Beim ersten, leichten Fixieren der Gegenelektrodeneinheit 20 auf der Haut durch die Operationsschwester kann sich beispielsweise ein Impedanzwert von 55 Ω zwischen den beiden Gegenelektroden 21, 22 ergeben. Die Auswerte- und Steuerschaltung 50 speichert den Wert von 55 Ω als momentanes Minimum ab. Nach dem anfänglichen leichten Fixieren wird die Operationsschwester die Gegenelektrodeneinheit 20 stärker an die Haut andrücken und beispielsweise mit einem Gurt festzurren. Dabei sinkt die gemessene Impedanz ab, beispielsweise auf einen Wert von etwa 42 Ω. Die Steuer- und Auswerteschaltung 50 speichert nun diesen niedrigeren Wert als momentanes Minimum ab. Liegt der Patient während der Behandlung nicht auf der Gegenelektrodeneinheit 20, so wird der gespeicherte Wert von 42 Ω im weiteren Verlauf der Operation in der Regel nicht mehr unterschritten wird. Wird dagegen der Patient für die Operation so gedreht, das er auf der Gegenelektrodeneinheit 20 liegt und somit sein Körpergewicht gegen die Gegenelektrodeneinheit 20 drückt, kann es sein, dass der bis dahin gespeicherte Minimalwert noch einmal unterschritten wird. In diesem Fall erfolgt nochmals eine Aktualisierung des gespeicherten Minimalwerts. Andernfalls kann davon ausgegangen werden, dass während der restlichen Behandlung das Minimum auf dem gespeicherten Wert von 42 Ω bleibt.

Auf Basis des gespeicherten Minimums legt die Auswerte- und Steuerschaltung 50 einen oder mehrere obere Grenzwerte fest, die zumindest während bestimmter Phasen des Behandlungsvorgangs nicht überschritten werden dürfen. Beispielsweise kann ein erster dynamischer Schwellenwert 15% über dem gespeicherten Minimalwert festgelegt werden und ein zweiter dynamischer Grenzwert 20% über dem gespeicherten Minimalwert. Da eine Bewegung eines Patienten während der HF-Abgabe nicht unmittelbar zu einer Fehlermeldung führen soll, kann während der HF-Abgabe als Kompromiss zwischen Sicherheit und Behandlungsziel der höhere Grenzwert zur Anwendung kommen. Der niedrigere Grenzwert, der nur eine 15%-ige Toleranz erlaubt, kann beispielsweise als Freigabeschwelle vor dem Einschalten der HF-Energie zur Anwendung kommen. Es hat sich gezeigt, dass sich bei Festlegung oberer Schwellen durch einen Aufschlag auf das gespeicherte Impedanzminimum um etwa 10% bis etwa 20% die Häufigkeit von Fehlalarmen oder Trivialalarmen in der Praxis deutlich reduzieren lässt.

Als statischer Grenzwert für die Impedanz zwischen den Gegenelektroden 21, 22 kann beispielsweise ein Wert von 80 Ω festgelegt sein, der deutlich über den für die dynamische Grenzwertkontrolle erwartbaren Schwellen liegt. Voraussetzung dafür, dass überhaupt eine HF-Abgabe stattfindet, ist, dass das gemessene Impedanzminimum und gegebenenfalls auch die hieraus errechneten dynamischen Grenzwerte unterhalb des statischen Grenzwerts liegen. Insoweit führt die Auswerte- und Steuerschaltung 50 eine Kombination von statischer und dynamischer Grenzwertkontrolle durch.

## Patentansprüche

1. Vorrichtung (10) für die Thermochirurgie, umfassend
- einen Generator (12) zur Bereitstellung hochfrequenter elektrischer Behandlungsenergie,
- eine Schnittstellenanschlussanordnung (14), welche den Anschluss einer Mehrzahl am oder im Körper eines Patienten (18) anzubringender Elektroden gestattet, wobei die Elektroden eine im Bereich des Behandlungsorts zu positionierende Anwendungselektrode (16) sowie mehrere flächige Gegenelektroden (21, 22, 26, 27) umfassen, und
- Impedanzmessmittel (30, 35, 40, 45; 36, 37, 45; 30, 41, 42), welche eine Impedanzmessung in mehreren Messkreisen gestatten, von denen jeder über mindestens eine an die Schnittstellenanschlussanordnung (14) angeschlossene flächige Gegenelektrode (21, 22, 26, 27) führt, wobei die Schnittstellenanschlussanordnung (14) den Anschluss mehrerer Gegenelektrodeneinheiten (20, 25) mit je zwei flächigen Gegenelektroden (21, 22, 26, 27) gestattet und die Messkreise in Zuordnung zu jeder Gegenelektrodeneinheit (20, 25) je einen Messkreis umfassen, in dem die flächigen Gegenelektroden (21, 22, 26, 27) der betreffenden Gegenelektrodeneinheit (20, 25) elektrisch in Reihe liegen, **dadurch gekennzeichnet, dass** die Messkreise mindestens einen Messkreis mit zwei elektrisch in Reihe liegenden Gegenelektrodeneinheiten (20, 25) umfassen, in dem die flächigen Gegenelektroden (21, 22) der einem Gegenelektrodeneinheit (20) elektrisch parallel zueinander liegen, und die flächigen Gegenelektroden (26,27) der anderen gegenelektrodeneinheit (25) elektrisch parallel zneinander liegen.

2. Vorrichtung nach Anspruch 1, **dadurch gekennzeichnet, dass** die Messkreise einen über die Anwendungselektrode (16) und mehrere, insbesondere alle flächigen Gegenelektroden (21, 22, 26, 27) führenden Messkreis umfassen, in dem die flächigen Gegenelektroden (21, 22, 26, 27) elektrisch parallel zueinander liegen.

3. Vorrichtung nach einem der vorhergehenden Ansprüche, **gekennzeichnet durch** eine Auswerte- und Steueranordnung (50), welche dazu ausgebildet ist, mindestens eine für die Impedanz in einem der Messkreise repräsentative Impedanzmessgröβe und/oder mindestens eine aus den Impedanzmessgrößen eines oder mehrerer Messkreise abgeleitete Größe zu überwachen und abhängig davon eine vorbestimmte Reaktion zu bewirken, dass wenigstens eine Impedanzmessgröße und/oder wenigstens eine abgeleitete Größe eine vorbestimmte Bedingung erfüllen.

4. Vorrichtung nach Anspruch 3, **dadurch gekennzeichnet, dass** die Auswerte- und Steueranordnung (50) dazu ausgebildet ist, die vorbestimmte Reaktion abhängig davon zu bewirken, dass eine Impedanzmessgröße und/oder eine abgeleitete Größe einen abhängig von wenigstens einem vorherigen Wert der Impedanzmessgröße bzw. der abgeleiteten Größe festgelegten Schwellenwert erreicht

5. Vorrichtung nach Anspruch 4, **dadurch gekennzeichnet, dass** die Auswerte- und Steueranordnung (50) dazu ausgebildet ist, den Schwellenwert abhängig von einem gemessenen Extremum der Impedanzmessgröβe bzw. der abgeleiteten Größe festzulegen.

6. Vorrichtung nach einem der Ansprüche 3 bis 5, **dadurch gekennzeichnet, dass** die Auswerte- und Steueranordnung (50) dazu ausgebildet ist, das Verhältnis der Impedanzmessgröβen zweier verschiedener Messkreise als eine abgeleitete Größe zu ermitteln.

7. Vorrichtung nach Anspruch 6, **dadurch gekennzeichnet, dass** die Auswerte- und Steueranordnung (50) dazu ausgebildet ist, die Impedanzmessgrößen zweier verschiedener Messkreise jeweils ins Verhältnis zur Impedanzmessgröβe eines weiteren Messkreises zu setzen und den zeitlichen Verlauf der so gebildeten abgeleiteten Größen im gegenseitigen Vergleich zu überwachen.

## Claims

1. A device (10) for thermosurgery, comprising
- a generator (12) for providing high frequency electrical treatment energy,
- an interface connection arrangement (14) which permits the connection of a plurality of electrodes to be placed on or in the body of a patient (18), the electrodes comprising an application electrode (16) to be positioned in the area of the treatment site and several planar counter-electrodes (21, 22, 26, 27), and
- impedance measurement means (30, 35, 40, 45; 36, 37, 45; 30, 41, 42) which permit impedance measurement in several measurement circuits, each of which passing via at least one planar counter-electrode (21, 22, 26, 27) which is connected to the interface connection arrangement (14),
wherein the interface connection arrangement (14) permits the connection of several counter-electrode units (20, 25) with two each planar counter-electrodes (21, 22, 26, 27) and the measurement circuits assigned to each counter-electrode unit (20, 25) comprise a measurement circuit each in which the planar counter-electrodes (21, 22, 26, 27) of the respective counter-electrode unit (20, 25) are electrically arranged in series,
**characterised in that** the measurement circuits comprise at least one measurement circuit with two counter-electrode units (20, 25) which are electrically arranged in series, in which the counter-electrodes (21, 22) of the one counter-electrode unit (20) are electrically arranged parallel to one another and the planar counter-electrodes (26, 27) of the other counter-electrode unit (25) are electrically arranged parallel to one another.

2. The device according to Claim 1, **characterised in that** the measurement circuits comprise a measurement circuit passing via the application electrode (16) and several, in particular all, planar counter-electrodes (21, 22, 26, 27), in which circuit the planar counter-electrodes, (21, 22, 26, 27) are electrically arranged parallel to one another.

3. The device according to one of the previous claims, **characterised by** an evaluation and control arrangement (50) which is adapted to monitor at least one impedance measured variable which is representative for the impedance in one of the measurement circuits and/or at least one variable which is derived from the impedance measured variables of one or several measurement circuits and to bring about a predetermined response depending on whether at least one impedance measured variable and/or at least one derived variable fulfils a predetermined condition.

4. The device according to Claim 3, **characterised in that** the evaluation and control arrangement (50) is adapted to bring about the predetermined response depending on whether an impedance measured variable and/or a derived variable reaches a threshold value which is set as a function of at least one previous value of the impedance measured variable or of the derived variable, respectively.

5. The device according to Claim 4, **characterised in that** the evaluation and control arrangement (50) is adapted to establish the threshold value as a function of a measured extreme value and the impedance measured variable or of the derived variable, respectively.

6. The device according to one of Claims 3 to 5, **characterised in that** the evaluation and control arrangement (50) is adapted to determine the ratio of the impedance measured variables of two different measurement circuits as a derived variable.

7. The device according to Claim 6, **characterised in that** the evaluation and control arrangement (50) is adapted to relate the impedance measured variables of two different measurement circuits to the impedance measured variable of another measurement circuit and to monitor the progress over time of the resultant derived variables in mutual comparisons.

## Revendications

1. Dispositif (10) de thermochirurgie, comprenant
- un générateur (12) destiné à fournir une énergie électrique de traitement à haute fréquence,
- un agencement de branchement d'interface (14) permettant le branchement de plusieurs électrodes à poser sur ou dans le corps d'un patient (18), ces électrodes comprenant une électrode d'application (16) à placer sur la zone à traiter et plusieurs contre-électrodes plates (21, 22, 26, 27), et
- des moyens de mesure d'impédance (30, 35, 40, 45 ; 36, 37, 45 ; 30, 41, 42) permettant une mesure d'impédance dans plusieurs circuits de mesure dont chacun passe par au moins une contre-électrode plate (21, 22, 26, 27) connectée audit agencement de branchement d'interface (14),
l'agencement de branchement d'interface (14) permettant le branchement de plusieurs unités de contre-électrodes (20, 25) avec respectivement deux contre-électrodes plates (21, 22, 26, 27), et les circuits de mesure, en association avec chaque unité de contre-électrodes (20, 25), comprenant chacun un circuit de mesure dans lequel les contre-électrodes plates (21, 22, 26, 27) de l'unité des contre-électrodes concernée (20, 25) sont montées électriquement en série,
**caractérisé en ce que** les circuits de mesure comprennent au moins un circuit de mesure avec deux unités de contre-électrodes (20, 25) montées électriquement en série, dans lequel les contre-électrodes plates (21, 22) de l'une des unités de contre-électrodes (20) sont montées électriquement en parallèle les unes par rapport aux autres, et les contre-électrodes plates (26, 27) de l'autre unité de contre-électrodes (25) sont montées électriquement en parallèle les unes par rapport aux autres.

2. Dispositif selon la revendication 1, **caractérisé en ce que** les circuits de mesure comprennent un circuit de mesure passant par ladite électrode d'application (16) et par plusieurs, en particulier toutes les contre-électrodes plates (21, 22, 26, 27), dans lequel les contre-électrodes plates (21, 22, 26, 27) sont montées électriquement en parallèle les unes par rapport aux autres.

3. Dispositif selon l'une des revendications précédentes, **caractérisé en ce qu'**un agencement de commande et d'évaluation (50), qui est conçu pour surveiller au moins une grandeur de mesure d'impédance représentative pour l'impédance dans un des circuits de mesure et/ou au moins une grandeur dérivée des grandeurs de mesure d'impédance d'un ou de plusieurs circuits de mesure, ainsi que pour provoquer en fonction de cela une réaction prédéterminée selon laquelle au moins une grandeur de mesure d'impédance et/ou au moins une grandeur dérivée remplissent une condition prédéterminée.

4. Dispositif selon la revendication 3, **caractérisé en ce que** l'agencement de commande et d'évaluation (50) est conçu pour provoquer ladite réaction prédéterminée en fonction de l'atteinte par une grandeur de mesure d'impédance et/ou une grandeur dérivée d'une valeur seuil fixée en fonction d'au moins une valeur antérieure de la grandeur de mesure d'impédance ou de la grandeur dérivée.

5. Dispositif selon la revendication 4, **caractérisé en ce que** l'agencement de commande et d'évaluation (50) est conçu pour fixer la valeur seuil en fonction d'une extrême mesurée de la grandeur de mesure d'impédance ou de la grandeur dérivée.

6. Dispositif selon l'une des revendications 3 à 5, **caractérisé en ce que** l'agencement de commande et d'évaluation (50) est conçu pour calculer sous forme de grandeur dérivée le rapport des grandeurs de mesure d'impédance de deux circuits de mesure différents.

7. Dispositif selon la revendication 6, **caractérisé en ce que** l'agencement de commande et d'évaluation (50) est conçu pour proportionner respectivement les grandeurs de mesure d'impédance de deux circuits de mesure différents à la grandeur de mesure d'impédance d'un circuit de mesure supplémentaire, et pour surveiller par comparaison réciproque l'évolution dans le temps des grandeurs dérivées ainsi obtenues.
